# FASCICULE DE BREVET EUROPEEN

(11) **EP 0 674 714 B1**
(45) Date de publication et mention de la délivrance du brevet: **10.12.2003**
(21) Numéro de dépôt: 94902826.0
(22) Date de dépôt: 16.12.1993
(51) Int. Cl.: C12N 15/55, C12N 1/21, C12N 11/00, C12N 9/20, A61K 38/00

(54) **LIPASE GASTRIQUE DU CHIEN RECOMBINANTE ET COMPOSITIONS PHARMACEUTIQUES**
REKOMBINANTE LIPASE AUS HUNDEMAGEN UND PHARMAZEUTISCHE ZUSAMMENSETZUNGEN
RECOMBINANT CANINE GASTRIC LIPASE AND PHARMACEUTICAL COMPOSITIONS

(30) Priorité: 16.12.1992 FR 9215201
(43) Date de publication de la demande: 04.10.1995
(73) Titulaire: MERISTEM THERAPEUTICS, 63100 Clermont-Ferrand (FR)
(72) Inventeur: BLANCHARD, Claire, F-91350 Grigny (FR); BENICOURT, Claude, F-78800 Houilles (FR); JUNIEN, Jean-Louis, F-92310 Sèvres (FR)
(74) Mandataire: Richebourg, Michel François
(86) Numéro de dépôt international: FR9301260
(87) Numéro de publication internationale: WO94013816

(56) Documents cités:
- EP-A- 0 261 016
- WO-A-86/01532
- DE-A- 3 737 333
- EUROPEAN JOURNAL OF BIOCHEMISTRY vol. 202 , 1991 pages 75 - 83 CARRIERE F. ET AL 'Purification and biochemical characterization of dog gastric lipase' cited in the application
- BIOCHIMICA ET BIOPHYSICA ACTA vol. 1083, no. 1 , 1991 pages 109 - 119 S. J. IVERSON ET AL 'Milk lipid digestion in the neonatal dog : the combined actions of gastric and bile salt stimulated lipases'

## Description

La présente invention concerne des acides nucléiques codant pour la lipase gastrique de chien (LGC), et autres dérivés polypeptidiques de cette dernière possèdant une activité lipasique, ainsi que leur utilisation, notamment pour la production de ces polypeptides. L'invention a également pour objet les polypeptides codés par ces acides nucléiques, et l'utilisation de ces polypeptides dans des compositions pharmaceutiques.

La LGC est une glycoprotéine d'environ 380 acides aminés (AA) d'un poids moléculaire d'environ 49 kilodaltons (KD) synthétisée sous forme d'un précurseur contenant un peptide signal à l'extrémité aminoterminale (NH₂-terminale) et sécrétée par les cellules médianes de la muqueuse fundique de l'estomac de chien (Carrière F. et al., Eur. J. Biochem. 202 (1991) 75-83).

Cette enzyme appartient à une famille de lipases dites "préduodénales" dont certains membres ont déjà été purifiés et parfois même clonés (Docherty A.J.P. et al., Nucl. Ac. res. 13 (1985) 1891-1903 ; Bodmer M.W. et al., Biochem. Biophys. Act. 909 (1987) 237-244; Moreau H. et al., Biochem. Biophys. Act. 960 (1988) 286-293; Brevets européens n° 0 191 061 et n° 0 261 016)

Pendant longtemps, on a tenu pour acquis que l'hydrolyse des lipides alimentaires s'effectuait au niveau de l'intestin grêle grâce à l'action d'enzymes produites par le pancréas (Bernard C., C. R. Acad. Sci. 28 (1849) 249-253).

Des observations laissaient cependant penser que l'hydrolyse des triglycérides pouvait avoir lieu dans l'estomac par le biais d'enzymes préduodénales (Volhard, F., Z. Klin. Med. 42 (1901) 414-429; Shonheyder, F. and Voiquartz, K. Acta Physiol. Scand. 9 (1945) 57-67). Ces enzymes, et en particulier la lipase gastrique de chien, ont des propriétés enzymatiques et physico-chimiques qui les différencient des lipases pancréatiques de mammifères. Ces différences entre lipases gastriques et pancréatiques concernent essentiellement les points suivants : poids moléculaire, composition en acides aminés, résistance à la pepsine, spécificité de substrat, pH optimum d'action, et stabilité en milieu acide.

De plus, in vitro, dans certaines conditions, on peut mettre en évidence une synergie d'action entre les lipases gastrique et pancréatique sur l'hydrolyse de triglycérides à chaînes longues (Gargouri, Y. et Al., Biochem. Biophys. Act. 1006 (1989) 255-271).

On connaît plusieurs situations pathologiques (mucoviscidose, insuffisance pancréatique exocrine) où les patients sont totalement ou partiellement dépourvus de sécrétion pancréatique exocrine et donc des enzymes nécessaires à l'hydrolyse des aliments (amylases, lipases, protéases). La non-absorption des graisses au niveau intestinal, et notamment des triglycérides à longues chaînes, se traduit par une augmentation très importante de la stéatorée chez ces patients et par un ralentissement très sensible de la prise de poids chez les jeunes malades. Pour remédier à cela on administre à ces sujets des extraits pancréatiques de porc au moment des repas. L'efficacité thérapeutique de ces extraits pourrait être nettement améliorée par la co-prescription de LGC grâce à sa spécificité d'action sur les triglycérides à longues chaînes.

Dans l'article paru dans Eur. J. Biochem. 201, 75-83, 1991, de F. Carrière sont décrites la purification et la détermination de la séquence NH₂-terminale de la LGC. Un procédé permettant d'extraire cette enzyme à partir d'estomacs de chien est également décrit dans cette publication. Ce procédé consiste essentiellement à faire subir à des estomacs de chiens une extraction par un milieu aqueux acide (pH 2,5), on relargue l'extrait lipasique par addition de sels hydrosolubles, puis par une filtration sur tamis moléculaire, suivie d'une séparation par chromatographies par échange d'ions, ainsi que par filtration sur gel, et l'on recueille une fraction d'élution contenant la lipase. La LGC purifiée obtenue par ces procédés a un poids moléculaire selon la technique de Laemmli de 49 000 daltons, dont 6 000 correspondent à des sucres et 43 000 à une protéine.

Des raisons évidentes de difficultés d'approvisionnement en estomacs de chiens empêchent tout développement de ce procédé tant au niveau du laboratoire qu'au niveau industriel. D'où la nécessité de trouver un procédé faisant abstraction de l'utilisation d'estomacs de chiens, qui permette de produire la LGC en grande quantité.

La présente invention a précisément pour but de permettre la production de LGC à l'échelle industrielle en supprimant tout problème d'approvisionnement en matière première, et à un prix de revient avantageux.

L'invention découle de la découverte faite par les inventeurs de la séquence nucléotidique de l'ARN messager (ARNm) codant pour la LGC, après clonage de l'ADN complémentaire (ADNc) de cet ARNm à l'aide d'une sonde correspondant à la séquence nucléotidique de la lipase gastrique recombinante de lapin décrite dans la demande de brevet français déposée le 13 novembre 1991, et publiée sous le numéro 2 683 549.

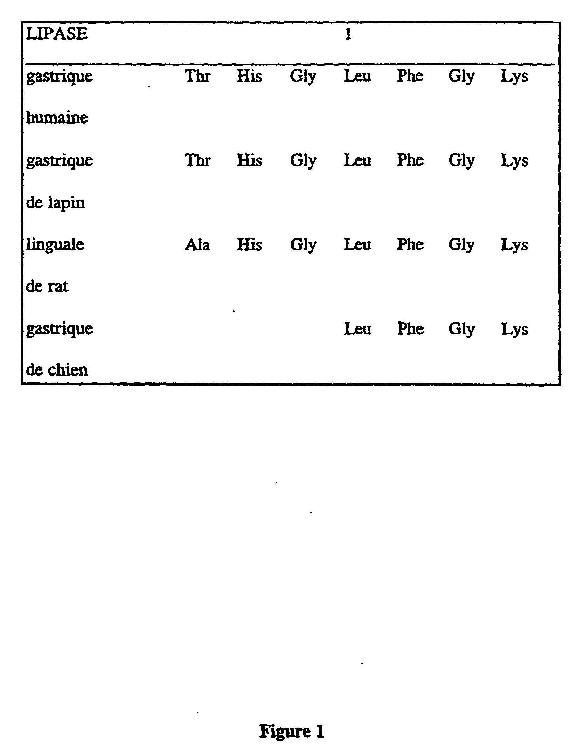

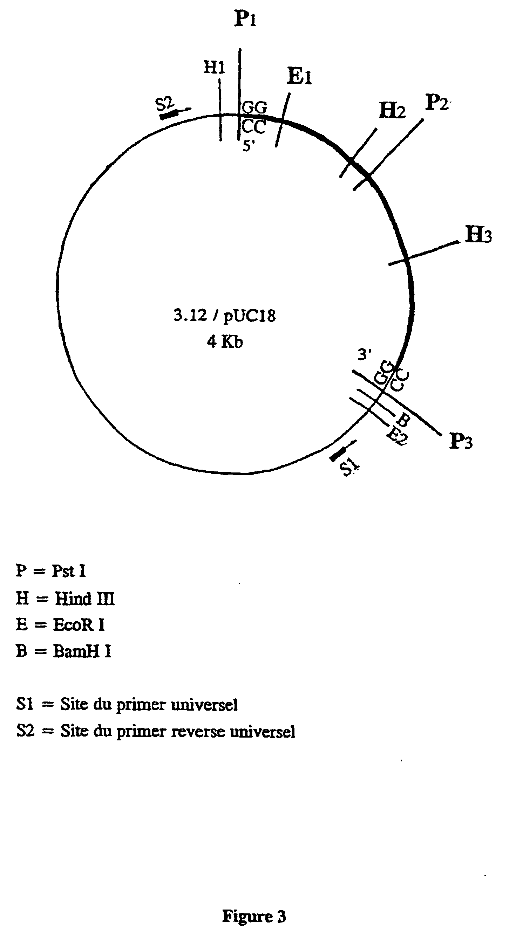

La présente invention sera plus particulièrement illustrée à l'aide des figures 1 à 12 dont les légendes sont les suivantes:
Figure 1: Séquences polypeptidiques de la région de coupure des précurseurs des lipases gastrique de lapin, homme et rat, et comparaison avec la séquence NH₂-terminale de la lipase gastrique de chien (SEQ ID NO 11).
Figure 2A: Conception d'un oligonucléotide dégénéré (DGL₁) codant pour la région de coupure du précurseur de la LGC à partir de la comparaison de ses homologues lapin, homme, rat.
Figure 2B: Séquence des oligonucléotides DGL₁ (SEQ ID NO 7) et LPC₂ (SEQ ID NO 8)
Figure 3: Cartographie du clone 3.12.
Figure 4: Schéma de clonage de la LGC dans le vecteur pBluescript KS(+) et sous clonage du fragment "H" du clone 3.12. dans pKSPCR: obtention du clone pKSDGL10.
Figure 5: Carte de restriction du vecteur plasmidique pRU303.
Figure 6: Séquence nucléotidique du fragment d'ADN EcoRI-NdeI du plasmide pRU303.
Figure7: Sous clonage de l'ADNc de la lipase gastrique de chien dans le vecteur d'expression pRU303 et construction du plasmide pDGL5.303.
Figure8: Séquence nucléotidique de l'ADNc codant pour la LGC mature (SEQ ID NO 1).
Figure 9A: Séquence polypeptidique de la LGC mature (SEQ ID NO 3).
Figure 9B: Comparaison des séquences polypeptidiques de la LGH (lipase gastrique humaine) et de la LGC, et détermination du % d'homologie.
Figure 9C: Comparaison des séquences polypeptidiques de la LLR (lipase linguale de rat) et de la LGC, et détermination du % d'homologie.
Figure 9D: Comparaison des séquences polypeptidiques de la LGL (lipase gastrique de lapin) et de la LGC, et détermination du % d'homologie.
Figure 10: Mutagénèse in vitro de l'ADNc de la LGC par la technique de "PCR" à l'aide d'amorces oligonucléotidiques DGL₂ (SEQ ID NO 9) et DGL₃ (SEQ ID NO 10) en vue de la construction du plasmide pDGL5.303.
Figure 11: Analyse par électrophorèse sur gel de polyacrylamide-SDS des protéines synthétisées, en absence ou en présence d'IPTG, dans E.coli W3110 I^{q} transformée par le plasmide pDGL5.303.
Figure 12: Immunodétection à l'aide d'anticorps spécifiques de la LGC synthétisée dans E. coli W3110 I^{q} transformée par le plasmide pDGL5.303 après transfert de type "Western" sur membrane de nylon des protéines issues de ces bactéries.

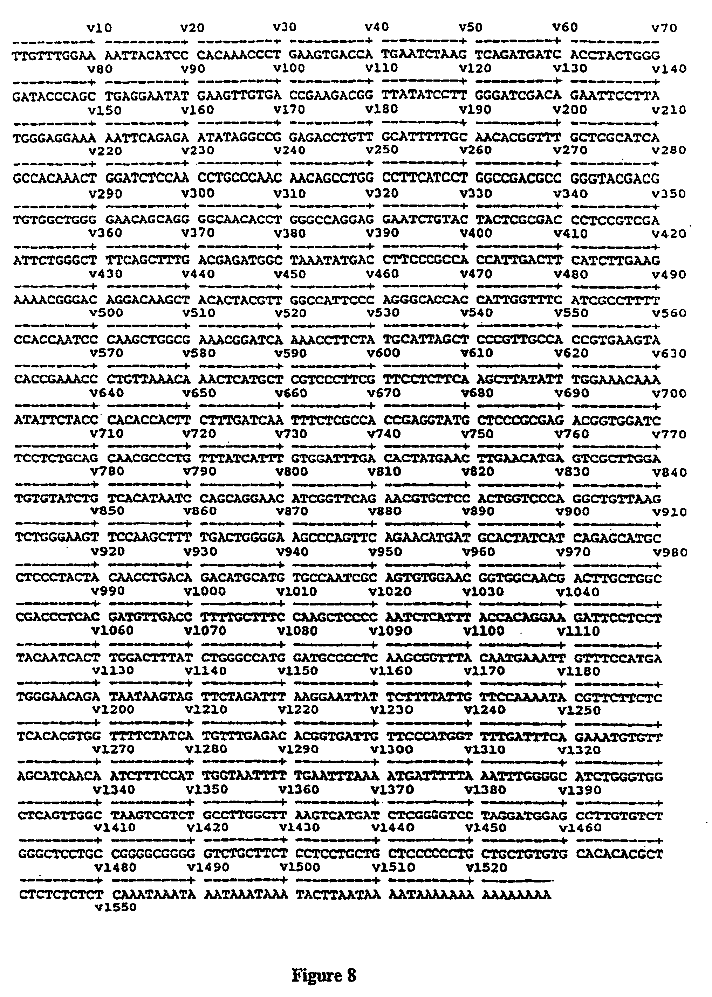

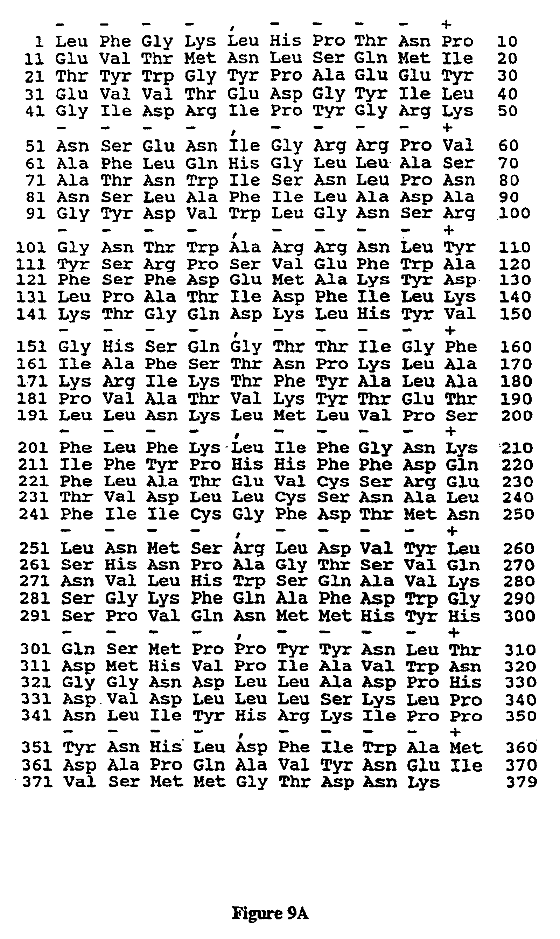

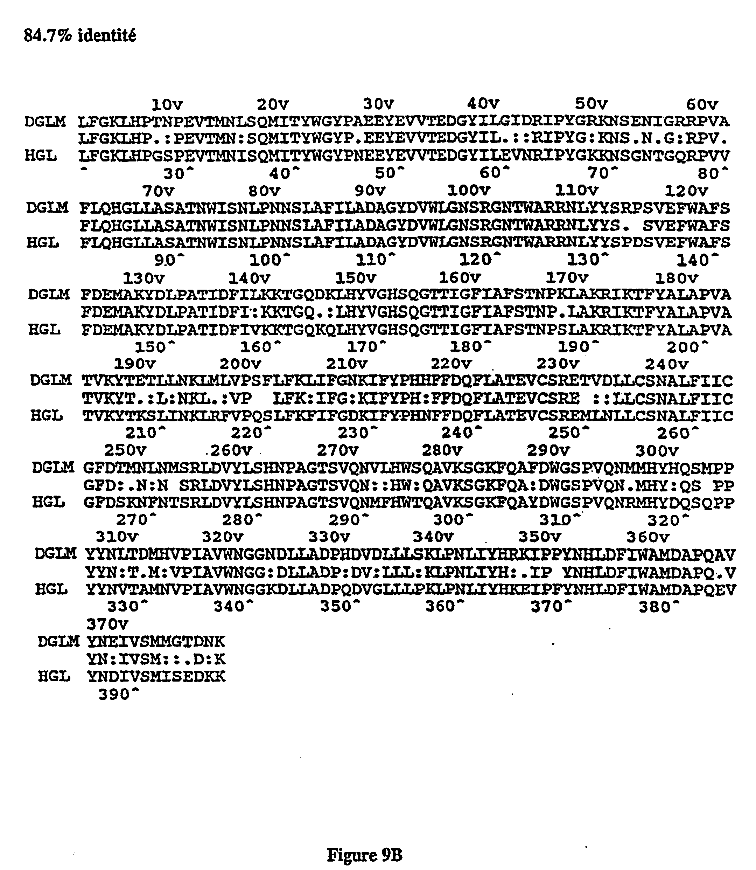

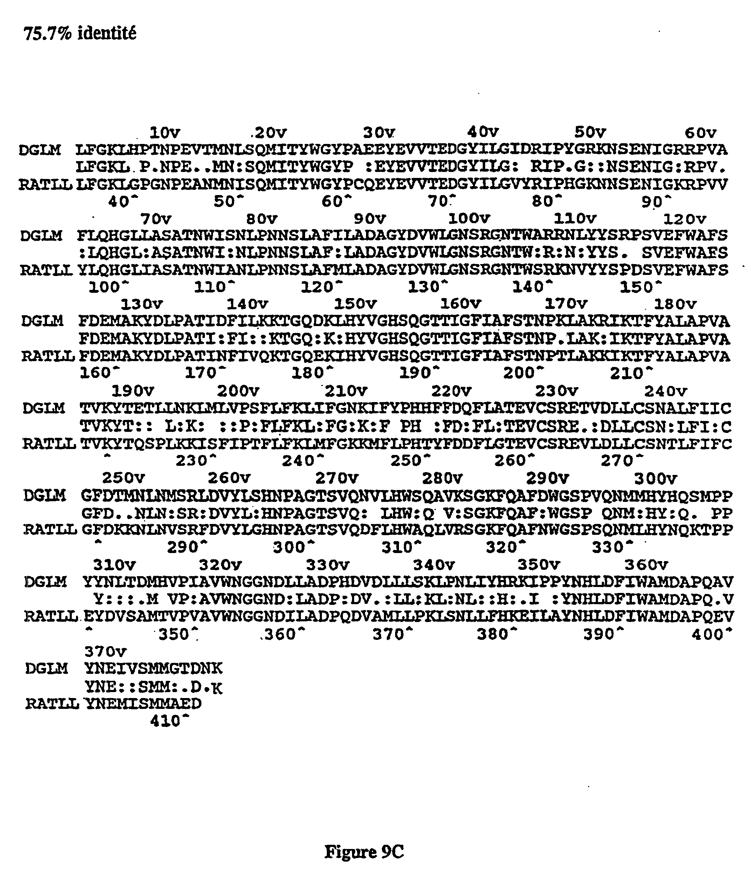

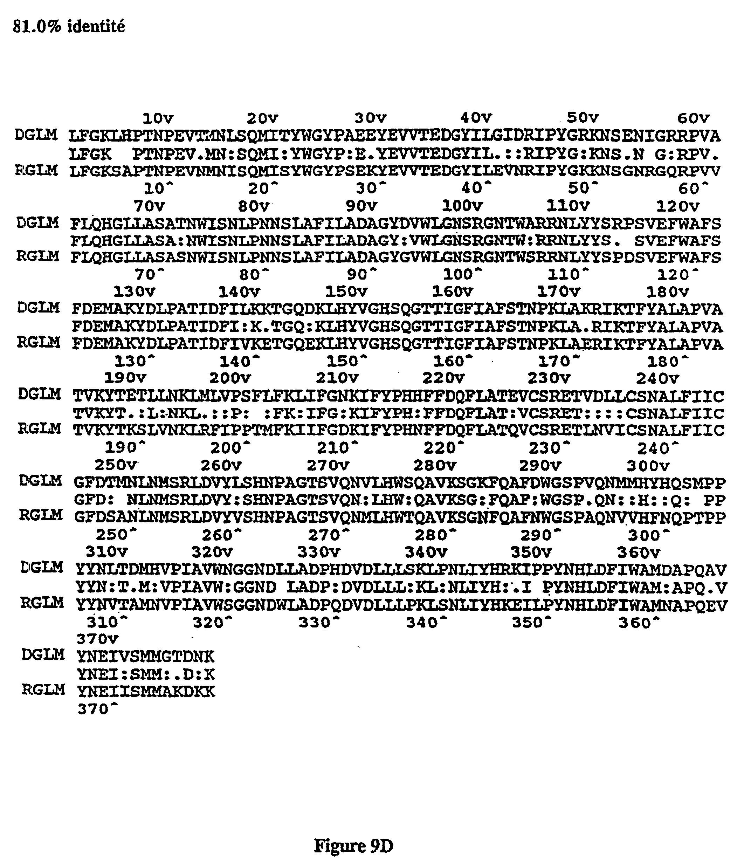

Ainsi la présente invention concerne tout acide nucléique caractérisé en ce qu'il comprend tout ou partie du fragment d'ADN représenté sur la figure 8 (SEQ ID NO 1), et plus particulièrement tout ou partie du fragment d' ADN délimité par les nucléotides situés aux positions 1 et 1137 (SEQ ID NO 2) de l'ADN représenté sur la figure 8, ce fragment d'ADN codant pour le polypeptide délimité par les acides aminés situés aux positions 1 et 379 de la séquence en acides aminés représentée sur la figure 9A (SEQ ID NO 3), ce polypeptide correspondant à la LGC mature.

Par l'expression LGC ci-dessus, et ci-après, on entend toute lipase sécrétée par la muqueuse gastrique ou par une muqueuse prégastrique chez le chien.

Les acides nucléiques susmentionnés peuvent également comprendre en amont du fragment d'ADN délimité par les nucléotides situés aux positions 1 et 1137, de la figure 8, un fragment d'ADN (plus particulièrement une séquence ATG) codant pour une méthionine (SEQ ID NO 4).

L'invention vise également les fragments d'ADN susmentionnés présentant en aval de la position 1137 un codon STOP, notamment celui constitué de la séquence délimitée par les nucléotides situés aux positions 1138, 1139 et 1140 de la figure 8 (SEQ ID NO 6).

La LGC, comme toutes les lipases gastriques purifiées ou clonées jusqu'à présent, est synthétisée sous forme d'un précurseur constitué d'un peptide signal précédant la séquence polypeptidique de la protéine mature.

D'une manière générale, l'invention concerne tout acide nucléique caractérisé en ce qu'il comprend en amont d'un des fragments d'ADN susmentionnés, une séquence nucléotidique codant pour un peptide signal.

Par opposition aux acides nucléiques double brin cités ci-dessus, l'invention vise également les acides nucléiques simple brin constitués de l'une ou l'autre des deux séquences nucléotidiques complémentaires constituant les fragments d'ADN susmentionnés.

L'invention concerne également tout acide nucléique susceptible de s'hybrider avec un acide nucléique simple brin tel que décrit ci-dessus, notamment dans les conditions d'hybridation citées dans la description détaillée qui suit du clonage de l'ADNc de la LGC selon l'invention.

Tout acide nucléique codant pour un polypeptide selon l'invention et dont la séquence nucléotidique diffère en fonction de la dégénérescence du code génétique des séquences nucléotidiques susmentionnées, entre également dans le cadre de la présente invention.

L' invention a également pour objet tout acide nucléique recombinant caractérisé en ce qu'il comprend un acide nucléique tel que décrit ci-dessus selon l'invention, inséré dans une molécule d' ADN hétérologue vis-à-vis du susdit acide nucléique.

A ce titre l'invention a plus particulièrement pour objet tout acide nucléique recombinant comprenant un promoteur situé en amont de l'acide nucléique selon l'invention, et sous le contrôle duquel la transcription dudit acide nucléique est susceptible d'être effectuée, ainsi qu'une séquence codant pour des signaux de terminaison de la transcription située en aval dudit acide nucléique.

L'invention concerne également tout vecteur recombinant, notamment du type plasmide, cosmide ou phage, caractérisé en ce qu'il contient un acide nucléique recombinant tel que décrit ci-dessus, inséré en l'un de ses sites non essentiels pour sa réplication.

Les vecteurs recombinants selon l'invention sont avantageusement caractérisés en ce qu'il contiennent en l'un de leurs sites non essentiels pour leur réplication, des éléments nécessaires pour promouvoir et contrôler l'expression d'un acide nucléique selon l'invention dans un hôte cellulaire, et plus particulièrement un promoteur reconnu par les polymérases de l'hôte cellulaire, notamment un promoteur inductible.

L'invention vise également tout hôte cellulaire, de type procaryote ou eucaryote, transformé par un vecteur recombinant tel que décrit ci-dessus, et comprenant les éléments de régulation permettant l'expression d'un gène ou d'un ADNc selon l'invention.

A titre d'exemples de cellules hôtes susceptibles d'être transformées par un vecteur recombinant selon l'invention, on peut citer les cellules de mammifères telles que les cellules COS ou CHO, des cellules d'insectes infectables par un virus recombinant de type baculovirus, les champignons filamenteux tels que *Aspergillus niger* ou *oryzae*, les levures telles que Saccharomyces cerevisiae ou Kluyveromyces lactis, ainsi que les bactéries telles que E.coli (bactérie Gram négatif) ou B.subtilis (bactérie Gram positif).

L'invention a également pour objet des amorces d'ADN (ou d'ARN) utilisables pour la synthèse d'acides nucléiques selon l'invention par la technique d'amplification en chaîne de l'ADN, ci-après désignée par technique PCR (Polymerase Chain Reaction). Cette technique est plus particulièrement décrite dans les brevets américains n° 4, 683, 202 et n° 4. 683, 195, ainsi que dans le brevet européen n° 200.362. Les amorces selon l'invention sont avantageusement constituées d'environ 15 à 40 nucléotides correspondant aux extrémités 3' et 5' de l'un et l'autre des deux brins constituant les fragments d'ADN susmentionnés.

L'invention vise également des sondes nucléotidiques issues de l'un ou l'autre des deux brins constituant les fragments d'ADN susmentionnés de l'invention, ainsi que l'utilisation de ces sondes, notamment pour la détection dans un échantillon biologique de la présence éventuelle de LGC.

Avantageusement, les sondes de l'invention sont constituées d'environ 17 à 23 nucléotides. La détection de la présence de LGC dans un échantillon, est de préférence réalisée après amplification du nombre de copies des gènes ou des ARNm codant pour la LGC susceptibles d'être présents dans cet échantillon, à l'aide des amorces indiquées ci-dessus.

A ce titre, l'invention concerne également un kit pour la mise en oeuvre de la méthode de détection susmentionnée, comprenant:
- le cas échéant, des amorces telles que décrites ci-dessus, ainsi que les réactifs pour la constitution d'un milieu propice à la réalisation de l'amplification de la séquence d'ADN ou d'ARN codant pour la LGC,
- une sonde nucléotidique telle que décrite ci-dessus, le cas échéant marquée, notamment de manière radioactive ou enzymatique, ainsi que les réactifs pour la constitution d'un milieu propice à la réalisation de l'hybridation entre la sonde et la séquence d'ADN ou d'ARN susmentionnée,
- les réactifs permettant la détection de la sonde hybridée avec ladite séquence.

Avantageusement les sondes nucléotidiques de l'invention sont suceptibles de s'hybrider à la fois avec la séquence d'ADN ou d'ARN codant pour la LGC et avec celles codant pour les lipases gastriques humaine (LGH) et de lapin (LGL). De telles sondes sont utilisables pour la mise en oeuvre d'une méthode de détection in vitro de la présence éventuelle de LGH dans un échantillon biologique susceptible de contenir cette dernière. Une telle méthode de détection est réalisée de la manière indiquée ci-dessus, et permet le diagnostic in vitro de pathologies liées à la surproduction, ou à l'inverse, à l'insuffisance, voire l'absence, de production de lipase gastrique dans l'organisme.

L'invention a également pour objet les polypeptides correspondant selon le code génétique universel aux acides nucléiques selon l'invention décrits ci-dessus, ou tout fragment de ces polypeptides recombinants, ou tout polypeptide modifié par substitution et/ou addition et/ou suppression d'un ou plusieurs acides aminés de ces polypeptides recombinants, ces fragments ou polypeptides modifiés conservant les propriétés enzymatiques des polypeptides recombinants susmentionnés.

Il faut entendre par "polypeptide recombinant" toute molécule possédant une chaîne polypeptidique susceptible d'être produite par voie du génie génétique, par transcription et traduction d'une séquence d'ADN correspondante sous le contrôle d'éléments de régulation appropriés à l'intérieur d'un hôte cellulaire efficace. Par conséquent, l'expression "polypeptides recombinants" n'exclut pas la possibilité que ces polypeptides aient subi des modifications pos-traductionnelles telles que la glycosylation.

Le terme "recombinant" implique le fait que le polypeptide a été produit par voie du génie génétique, plus particulièrement en raison du fait que ce polypeptide résulte de l'expression dans un hôte cellulaire de séquences d'acides nucléiques qui ont été préalablement introduites dans un vecteur d'expression utilisé dans ledit hôte.

Toutefois, il doit être entendu que cette expression n'exclut pas la possibilité que le polypeptide soit produit par un procédé différent, par exemple par synthèse chimique classique ;selon les méthodes classiquement utilisées pour la synthèse des protéines, ou par clivage de molécules de plus grande taille.

L'invention vise également les polypeptides susmentionnés sous forme biologiquement pure. Il faut entendre par l'expression "biologiquement pure" d'une part un degré de pureté permettant au polypeptide recombinant d'être utilisé pour la production de compositions pharmaceutiques, et, d'autre part, l'absence de contaminants, plus particulièrement de contaminants naturels.

A ce titre, l'invention concerne plus particulièrement:
- le polypeptide délimité par les acides aminés situés aux positions 1 et 379 de la séquence en acides aminés représentée sur la figure 9A (SEQ ID NO 3), et correspondant à la LGC mature telle qu'obtenue par voie du génie génétique, et dont le poids moléculaire varie d'environ 43.200 à environ 50.000 daltons, suivant que l'hôte, dans lequel elle est produite, effectue des modifications post-traductionnelles sur la chaîne polypeptidique de cette LGC,
- les polypeptides susmentionnés dont les séquences en acides aminés sont précédées d'une méthionine (SEQ ID NO 5).

Avantageusement, les polypeptides susmentionnés selon l'invention, et plus particulièrement la LGC recombinante, possèdent une activité lipolytique comprise entre environ 50 U/mg de polypeptide et environ 750 U/mg de polypeptide, et de préférence supérieure à 250 U/mg de polypeptide lorsque mesurée à l'aide d'un triglycéride à chaîne courte (tel que la tributyrine), comme substrat selon la méthode de Gargouri (plus particulièrement décrite dans la description détaillée qui suit de l'invention). Une unité U correspond à la quantité d'enzyme nécessaire pour libérer une µmole d'ions H⁺ (c'est-à-dire, d'acides gras libres) par minute à 37°C.

L'activité lipolytique maximale des polypeptides recombinants, selon l'invention, sur les acides gras à chaîne longue, est avantageusement obtenue à des valeurs de pH de 3 à 5.

Selon un autre aspect avantageux des polypeptides recombinants de l'invention, leur activité lipolytique reste inchangée après incubation durant une heure à pH 2 et à 37°C.

La présente invention concerne également un procédé de préparation d'un polypeptide tel que décrit ci-dessus, ce procédé comprenant la succession d'étapes suivantes:
- la mise en culture d'un hôte cellulaire, transformé par un vecteur recombinant tel que décrit ci-dessus, dans un milieu de culture approprié, et
- la récupération du polypeptide produit par ledit hôte cellulaire, soit directement à partir du susdit milieu de culture, lorsque la séquence codant pour ledit polypeptide est précédée d'une séquence signal et que l'hôte cellulaire est capable de sécréter le polypeptide dans le milieu de culture (notamment dans le cas des cellules eucaryotes et des levures), soit après lyse de l'hôte cellulaire (notamment dans le cas de bactéries).

Le cas échéant, l'étape de récupération est suivie d'une étape de purification du polypeptide récupéré, et notamment après récupération par lyse de la bactérie par une étape de solubilisation du polypeptide, puis de sa renaturation.

Les agents et les techniques de solubilisation des polypeptides obtenus sous forme d'inclusion sont bien connus de l'homme de l'art. Essentiellement les agents solubilisants sont l'urée, des halogénures d'ammonium quaternaires comme le chlorure de guanidinium ou le chlorure de cétyltriméthylammonium qui sont utilisés dans des procédures expérimentales telles que celles décrites par N.K. Puri et coll. dans Biochem. J (1992) 285, 871-879.

Avantageusement, comme il l'a été déjà précisé plus haut, les séquences nucléotidiques codant pour les polypeptides dont la production est recherchée, et insérées dans le vecteur utilisé pour transformer les cellules hôtes, sont précédées d'une séquence signal permettant ainsi la sécrétion des polypeptides produits hors des cellules hôtes et leur recupération directement à partir du milieu de culture sans avoir à procéder à la lyse desdites cellules hôtes.

A titre d'exemple, la synthèse de la LGC mature dans des cellules de mammifères telles que les cellules COS ou les cellules CHO pourra être obtenue en insérant l'acide nucléique codant pour le précurseur de la LGC dans un vecteur d'expression approprié.

La présence du segment d'ADN codant pour le peptide signal permettra à la machinerie cellulaire de glycosyler dans le réticulum endoplasmisque et de sécréter la LGC dans le milieu de culture sous forme biologiquement active.

Alternativement, la production de lipase gastrique de chien par des cellules d'insectes pourra être obtenue en insérant l'ADNc codant pour la LGC ou son précurseur derrière un promoteur approprié dans le génome d'un virus de type baculovirus susceptible d'infecter lesdites cellules.

Pour faire produire et sécréter la LGC par une levure telle que Saccharomyces cerevisiae ou Kluyveromyces lactis, il sera préférable de remplacer, dans le cDNA, le segment d'ADN codant pour le peptide signal de la LGC par un fragment d'ADN codant pour un peptide signal de protéine de levure. L'ADNc recombinant ainsi obtenu sera alors introduit dans un vecteur d'expression spécifique de l'hôte considéré. De tels systèmes d'expression sont maintenant relativement courants. Par exemple, on peut citer l'expression de la sérum albumine humaine (Brevet européen n° 0361 991 A2) ou de la chymosine de veau (Van den Berg J.A. et al., Biotechnology 8 (1990) 135-139).

Escherichia coli est une bactérie Gram négatif pourvue d'une paroi chez qui les phénomènes de sécrétion de protéines dans le milieu de culture sont extrêmement réduits. Un certain nombre de protéines s'accumulent dans le périplasme bactérien grâce à la présence de signaux similaires aux séquences signal des protéines eucaryotes. Parmi celles-ci, on peut citer les produits des gènes phoA et malE par exemple. Certaines régions de ces gènes ont été utilisées pour produire des protéines hétérologues dans l'espace périplasmique de E.coli. Cependant, la synthèse dans le cytoplasme de protéines étrangères demeure le système le mieux connu et le plus utilisé chez E. coli.

Le respect de certaines règles déduites de l'expérience lors de la réalisation des constructions de plasmides permet d'optimiser le niveau d'expression des protéines d'intérêt.

Dans un premier temps, il conviendra de placer l'ADNc codant pour la partie mature de la LGC, c'est-à-dire dépourvu du segment codant pour le peptide signal, derrière un promoteur bactérien ou phagique puissant. Pour éviter des problèmes de toxicité éventuelle de la protéine étrangère dans la bactérie, on choisira de préférence un promoteur inductible par un agent chimique (promoteurs Lac ou Trp) ou par un agent physique tel que le changement de température (promoteur P_{L} et répresseur cI857). L'ADNc devra être contigu, dans sa région 5' terminale à une séquence ATG spécifiant l'initiation de la synthèse protéique sur l'ARN messager. Cet ATG initiateur devra être précédé, à une distance de 6 à 12 paires de bases, d'une région riche en purines, appelée région de Shine et Dalgarno, et correspondant, sur l'ARN messager, au site de fixation des ribosomes.

La séquence du segment d'ADN situé entre la région de Shine et Dalgarno et l'ATG initiateur pourra être modifiée dans sa composition de manière à réduire les éléments de structure secondaire autour du codon d'initiation AUG sur l'ARN messager. Une fois les modifications nécessaires apportées, le vecteur sera introduit dans un hôte approprié.

L'invention concerne les anticorps dirigés contre les polypeptides de l'invention, et plus particulièrement ceux dirigés contre la LGC et susceptibles de reconnaître également la LGH et la LGL. De tels anticorps peuvent être obtenus par immunisation d'un animal avec ces polypeptides suivie de la récupération des anticorps formés.

Il va de soi que cette production n'est pas limitée aux anticorps polyclonaux.

Elle s'applique encore à tout anticorps monoclonal produit par tout hybridome susceptible d'être formé, par des méthodes classiques, à partir des cellules spléniques d'un animal, notamment de souris ou de rat, immunisés contre l'un des polypeptides purifiés de l'invention d'une part, et des cellules d'un myélome approprié d'autre part, et d'être sélectionné, par sa capacité à produire des anticorps monoclonaux reconnaissant le polypeptide initialement mis en oeuvre pour l'immunisation des animaux, ainsi que la LGH.

L'invention vise également l'utilisation de ces anticorps pour la mise en oeuvre d'une méthode de détection ou de dosage de la LGC ou de la LGH dans un échantillon biologique susceptible de la contenir.

L'invention concerne plus particulièrement l'utilisation de ces anticorps pour la mise en oeuvre d'une méthode de diagnostic in vitro de pathologies liées à la surproduction, ou à l'inverse, à l'insuffisance, voire l'absence de production de lipase dans l'organisme.

Cette méthode de diagnostic in vitro, réalisée à partir d'un échantillon biologique prélevé chez un patient, comprend une étape de mise en présence de cet échantillon, suivie d'une étape de détection des éventuels complexes anticorps-LGH formés lors de l'étape précédente.

A ce titre l'invention concerne également un kit pour la mise en oeuvre d'une méthode de détection ou de diagnostic in vitro susmentionnée, comprenant:
- des anticorps tels que décrits ci-dessus, avantageusement marqués de manière radioactive ou enzymatique, ainsi que les réactifs pour la constitution d'un milieu propice à la réalisation de la réaction immunologique entre ces anticorps et la LGH,
- les réactifs permettant la détection des complexes immunologiques formés entre ces anticorps et la LGH.

L'invention concerne également l'utilisation d'un ou plusieurs polypeptides décrits ci-dessus, pour l'obtention de compositions pharmaceutiques, utilisables notamment par voie orale, destinées à faciliter l'absorption des graisses animales ou végétales ingérées par un individu sain ou atteint d'une ou plusieurs pathologies affectant ou non le taux de production de lipase gastrique. Notamment, de telles compositions sont avantageusement utilisées chez les individus subissant un traitement médical altérant le mécanisme d'absorption des graisses, ou encore chez les personnes agées.

L'invention vise plus particulièrement l'utilisation d'un, ou plusieurs polypeptides décrits ci-dessus pour l'obtention de médicaments destinés au traitement des pathologies liées à l'insuffisance, voire l'absence, de production de lipases dans l'organisme, et plus particulièrement de pathologies telles que la mucoviscidose, et l'insuffisance pancréatique exocrine.

L'invention a également pour objet des compositions pharmaceutiques comprenant au moins un polypeptide selon l'invention, le cas échéant en combinaison avec un ou plusieurs autres polypeptides à activité lipasique, en association avec un véhicule pharmaceutiquement acceptable.

Les compositions pharmaceutiques selon l'invention sont de préférence administrables par voie orale, et se présentent notamment sous forme de gélules, de comprimés ou de poudres à diluer.

La posologie journalière chez l'homme est avantageusement d'environ 400 mg à environ 1 200 mg, répartis de préférence au moment des repas principaux, soit à raison d'environ 130 mg à environ 400 mg par repas.

L'invention concerne également l'utilisation des polypeptides tels que décrits ci-dessus selon l'invention pour la mise en oeuvre de réactions de bioconversions enzymatiques (telles que hydrolyses, trans-estérifications enzymatiques), notamment sous forme immobilisée sur un support solide.

S'agissant de la préparation des acides nucléiques de l'invention, celle-ci peut être effectuée par voie chimique, notamment selon l'un des procédés suivants.

Un mode de préparation approprié des acides nucléiques (comportant au maximum 200 nucléotides) de l'invention par voie chimique comprend les étapes suivantes:
- la synthèse d'ADN en utilisant la méthode automatisée de β-cyanéthyl phosphoramidite décrite dans Bioorganic Chemistry 4; 274-325, 1986,
- le clonage des ADN ainsi obtenus dans un vecteur plasmidien approprié et la récupération des ADN par hybridation avec une sonde appropriée.

Un mode de préparation, par voie chimique, d'acides nucléiques de longueur supérieure à 200 nucléotides, comprend les étapes suivantes:
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Nat. Acad. Sci. USA 80; 7461-7465, 1983,
- le clonage des ADN ainsi obtenus dans un vecteur plasmidien approprié et la récupération de l'acide nucléique recherché par hybridation avec une sonde appropriée.

L'invention sera plus particulièrement illustrée à l'aide de la description détaillée qui suit de la construction de vecteurs recombinants selon l'invention et de leur utilisation pour la production de LGC.

Une préparation d'ARN a été réalisée à partir de muqueuse isolée de la région fundique d'estomac de chien. Les ARN messagers isolés par chromatographie d'affinité, sur colonne d'oligo-dT cellulose ont été convertis en ADN complémentaire (ADNc) grâce à l'utilisation d'enzymes particulières: la reverse transcriptase du Virus du Sarcome de Rous et la DNA polymérase I de E.coli (fragment de Klenow). Cet ADNc a été introduit dans le vecteur pUC18 après certaines modifications et les molécules recombinantes ont été utilisées pour transformer la bactérie E.coli MM294. Les clones transformants ont été criblés par hybridation in situ à l'aide d'une sonde contenant l'ADNc de la lipase gastrique de lapin marquée radioactivement. Après autoradiographie, les colonies bactériennes correspondant à un signal positif lors de l'expérience d'hybridation, ont été isolées et l'ADN plasmidique présent dans leur cytoplasme amplifié et purifié.

Après criblage des clones obtenus, le clone 3.12 a été sélectionné et séquencé. Ce clone contient un insert PstI-PstI de 1201 paires de bases, insert lui-même divisé en deux parties inégales H et L par un site de restriction PstI.

Aucun clone contenant l'ADNc complet n'a pu être mis en évidence à ce stade.

Afin d'isoler le clone contenant l'ADNc codant pour la lipase de chien mature, une technique complémentaire a été utilisée.

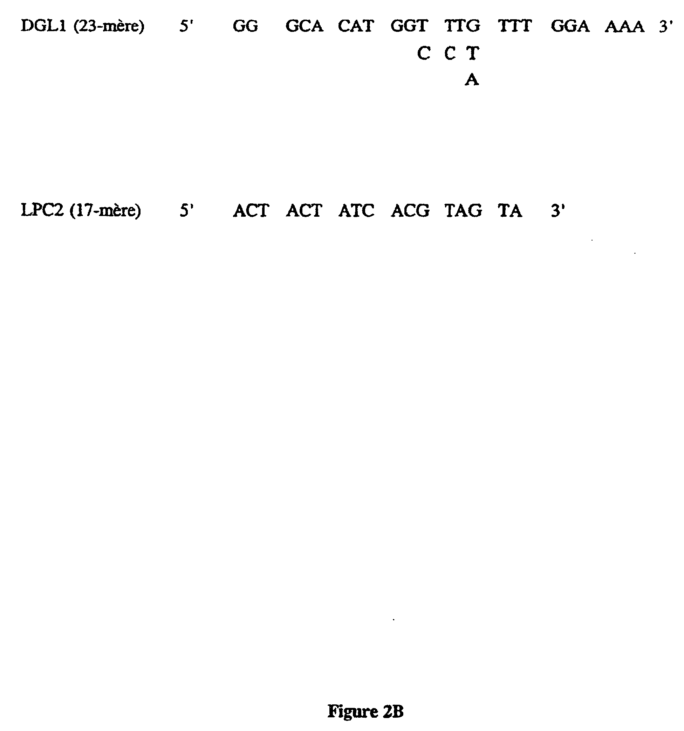

Une fraction d'ARNm issue de la préparation de départ est convertie en ADNc simple brin grâce à l'enzyme reverse transcriptase et à une amorce oligonucléotidique LPC₂ (figure 2B) obtenue à partir de la séquence 3' terminale de l'ADNc contenu dans le clone 3.12 précédemment isolé et sequencé.

L'ADNc codant pour la partie mature de la LGC est ensuite obtenu et amplifié par la méthode de PCR, en présence de Taq Polymérase et de deux amorces oligonucléotidiques, LPC₂ telle que susmentionnée, et DGL₁ conçue à partir de la comparaison des séquences nucléotidiques 5'terminales des lipases gastriques humaine et de lapin, de la lipase linguale de rat et de la séquence protéique NH₂-terminale connue de la LGC.

L'ADNc double brin ainsi obtenu a été introduit dans le vecteur pBluescript KS(+) après certaines modifications et les molécules recombinantes ont été utilisées pour transformer la bactérie E. coli MM294. Les clones transformants ont été criblés par PCR à l'aide de sondes oligonucléotidiques correspondant aux parties de la séquence du vecteur pBluescript KS(+) situées de part et d'autre de l'insert. Le clone pKSPCR contenant un insert de 700 paires de bases a été sélectionné et séquencé.

Parallèlement, après digestion par l'enzyme de restriction PstI, le fragment "H" de l'insert d'ADNc du clone 3.12 tel qu'obtenu précédemment est inséré dans le plasmide pKSPCR linéarisé par PstI; on obtient un clone pKSPCR 10 qui contient l'ADNc codant pour la lipase gastrique de chien mature.

L'analyse de la séquence nucléotidique de cet ADNc a permis de mettre en évidence une phase ouverte de lecture de 1137 nucléotides (NT) correspondant à une protéine de 379 AA et d'un poids moléculaire de 43222 daltons.

La comparaison avec les séquences nucléotidiques des autres lipases préduodénales (Docherty, A.P.J. et al. (1985) op.cit.; Bodmer, M.W. et al. (1987) op.cit.; Moreau, H. et al (1988) op.cit.) fait apparaître une homologie de 84,7 % avec la LGH, de 75,7 % avec la LLR et de 81 % avec la LGL dans les régions codantes.

De façon alternative, un second procédé peut être utilisé pour l'obtention d'un clone contenant l'ADNc codant pour la LGC mature.

Dans le cas où les ARNm extraits de la muqueuse d'estomac de chien, isolés par chromatographie d'affinité sur colonne oligo-dT, et convertis en ADNc grâce à l'utilisation d'enzymes spécifiques (reverse transcriptase du virus du Sarcome de Rous et DNA polymérase I de E.coli) correspondent à l'ARNm entier de la LGC mature ou de son précurseur, l'ADNc ainsi obtenu pourra être introduit après certaines modifications dans le vecteur pUC18 et les molécules recombinantes utilisées pour transformer une cellule hôte, de préférence bactérie ou levure; les clones transformants seront criblés par hybridation in situ à l'aide de sondes issues de la lipase gastrique de lapin.

Après autoradiographie, les colonies de cellules hôtes correspondant à un signal positif lors de l'hybridation seront isolées et l'ADN plasmidique présent dans le cytoplasme de ces cellules, amplifié et purifié. Avantageusement, les techniques générales de clonage utilisées dans ce second procédé seront les mêmes que celles mises en oeuvre dans le procédé précédemment décrit.

### Techniques générales de clonage:

Les méthodes classiques de biologie moléculaire telles que la purification des ARN messagers, l'extraction et la purification d'ADN plasmidique, la digestion par des enzymes de restriction, l'électrophorèse sur gel d'agarose ou de polyacrylamide, l'électroélution de gel d'agarose de fragments d'ADN, la transformation dans E.coli, sont décrites dans la littérature (Maniatis, T. et al., "Molecular cloning: a laboratory manual, Second Edition", Cold Spring Harbor Laboratory, Cold Spring Harbor, N.Y., 1989 ; Ausubel, F.M. et al. (eds.), "Current Protocols in Molecular Biology", John Willey and Sons, New-York, 1987).

Le "random priming" s'effectue selon la méthode décrite par Feinberg et Wogelstein (Anal. Biochem. (1983) 132: 6; Anal. Biochem. (1984) 137: 266).

Les enzymes sont obtenues auprès des sociétés Boehringer ou New England Biolabs et utilisées dans les conditions préconisées par les fournisseurs.

Les fragments d'ADN destinés à être assemblés sont séparés selon leur taille par électrophorèse sur gel d'agarose 1 %, purifiés par électroélution et précipités par l'éthanol. La ligature des fragments d'ADN s'effectue en présence de T₄DNA ligase à 4°C ou à 16°C dans un tampon approprié selon que les morceaux à assembler possèdent des extrémités franches ou cohésives.

Le séquençage de l'ADN s'effectue selon la méthode des didésoxynucléotides (Sanger, F. et al., Proc. Natl. Acad. Sci. USA. 74 (1977) (5463-5467) en utilisant un kit "T7 Sequencing" (Pharmacia).

L'amplification enzymatique de fragments d'ADN spécifiques s'effectue selon la méthode de "Polymerase-catalysed Chain Reaction" ou PCR (Saiki, R.K. et al., Science 220 (1985) 1350-1354) à l'aide d'un appareil PREM III LEP Scientific.

Les oligonucléotides utilisés comme amorces dans les réactions de PCR ou de séquençage sont synthétisés à l'aide d'un synthétiseur d'ADN modèle 391 PCR-MATE (Applied Biosystems) et purifiés par chromatographie haute pression en phase liquide avant leur utilisation.

Les molécules d'ADN recombinantes sont utilisées pour transformer des cellules compétentes des souches suivantes de E. coli :
- MM294 [F-, endA1, hsdR17 (rₖ-mₖ+), supE44, thi-1, relA1], ou
- W3110 I^{q}[F'TraD36, LacI^{q}, _(lac Z)M15, pro+].

L'ADN plasmidique est extrait des transformants bactériens résistants à l'ampicilline selon un protocole dérivé de la méthode de lyse alcaline décrite par Birnboin et Doly (Birnboim, H.C. and Doly, J., Nucl. Ac. Res. 7 (1979) 1512-1523).

L'immunodétection de la lipase gastrique de chien synthétisée dans la bactérie E. coli W3110 I^{q}, après addition d'IsoPropylThioGalactopyranoside (IPTG) au milieu de culture, s'effectue par une méthode d'immunoempreinte sur membrane de nylon en utilisant un anticorps de cobaye anti-LGC et le kit de révélation à la peroxydase ImmunoPure ABC (Pierce).

La préparation de la LGC est avantageusement illustrée, quoique de façon non limitative, par l'exemple suivant de l'expression de la LGC dans la bactérie E. coli W3110 I^{q}.

Le procédé de préparation de la lipase comprend plusieurs étapes qui sont détaillées dans ce qui suit:

### Etape n° 1: Clonage d'un ADNc codant pour la lipase gastrique de chien.

### 1.1. Isolement et purification des ARN messagers de muqueuse fundique d'estomac de chien.

Après broyage des tissus dans un tampon contenant du chlorure de lithium et de l'urée (Auffray, C. and Rougeon, F., Eur. J. Biochem. 107 (1980) 303-314), l'ARN total est séparé de l'ADN par précipitation sélective au chlorure de lithium. Les protéines contaminant l'ARN sont alors éliminées par extraction phénolique. Les ARN messagers, polyadénylés à leur extrémité 3'OH, sont séparés des ARN ribosomaux par chromatographie sur colonne d'oligo-dT cellulose (Maniatis, T. et al., déjà cité). On obtient ainsi environ 75 microgrammes d'ARN messager par gramme de tissu.

### 1.2. Mise en évidence de l'ARN messager codant pour la LGC dans la préparation d'ARN messager extraite de muqueuse fundique d'estomac de chien.

La lipase gastrique de chien a été purifiée à l'homogénéité et sa séquence polypeptidique NH₂-terminale déterminée (Carrière F. et al, déjà cité).

Une sonde constituée de l'ADN codant pour le précurseur de la lipase de lapin est utilisée pour vérifier la présence d'un ARNm codant pour la LGC dans la préparation obtenue.

Une expérience d'hybridation de type "Northern" est réalisée. Un échantillon de 20 µg d'ARN messager d'estomac de chien est dénaturé à 60° C, en présence de glyoxal et de DMSO, puis les ARNm sont séparés selon la taille, par électrophorèse, dans un gel d'agarose 1% en tampon phosphate 10mM pH=7 (Thomas, P., Proc. Natl. Acad. Sci. USA. 77 (1980) 5201-5205).

Après électrophorèse, l'ARN messager est transféré sur une membrane de nylon (Biodyne PALL) selon le protocole préconisé par le fournisseur.

Le fragment d'ADNc correspondant à la lipase de lapin est marqué par "random priming". Les membranes précédemment obtenues sont hybridées individuellement pendant 36 heures à37°C dans un tampon 5 X SSC -5 X Denhardt's - 50 mM phosphate de sodium pH=6,5 - 0,1% SDS - 50% formamide, contenant 10 ng/ml de la sonde radioactive (Ausubel, F. et al. (eds), déjà cité). Les températures utilisées tiennent compte des éventuelles homologies de séquences entre la LGL et la LGC. Un ARNm d'environ 1700 nucléotides s'hybride avec la sonde radioactive.

### 1.3. Synthèse d'ADN complémentaire à partir d'ARNm d'estomac de chien et insertion dans le vecteur pUC18.

La synthèse d'ADNc double brin est réalisée à partir de 4µg d'ARN polyA+, en présence de 50 unités d'AMV réverse transcriptase, de 100 ng d'une amorce oligo-dT et de la DNA polymérase I de E.coli.

Une fraction de cet ADN est inséré dans le vecteur pUC18 par le biais de queues oligo-dC et oligo-dG, ajoutées respectivement sur l'ADNc et sur le vecteur préalablement linéarisé par l'enzyme PstI (Gubler,U. and Hoffman, B.J., Gene 25 (1983) 263-269).

Les molécules hybrides sont utilisées pour transformer des bactéries compétentes E.coli MM294. La sélection des transformants s'effectue en étalant le produit de la transformation sur un milieu nutritif solide (LB-Agar) contenant de l'ampicilline à 50 mg/litre.

### 1.4. Isolement de l'ADNc codant pour la LGC.

Les colonies bactériennes issues de la transformation, sont tranférées sur des membranes de nylon (Biodyne PALL) et lysées selon un procédé recommandé par le fournisseur. Cette opération a pour effet de dénaturer et de fixer sur la membrane l'ADN bactérien et plasmidique contenu dans les colonies.

Après plusieurs lavages dans un tampon 3X SSC - 0,1 % SDS, à température ambiante puis à 65°C, les filtres sont préhybridés pendant deux heures à 65°C dans un tampon 6 X SSC - 10 X Denhardt's - 0,1 % SDS, puis hybridées à 50°C dans le même tampon contenant la sonde lapin marquée au ³²P par "random priming", à raison de 0,5 µci par ml de tampon.

Les filtres sont lavés dans un tampon 2 X SSC - 0,1 % SDS à température ambiante puis à 50°C dans le même tampon, avant d'être placés en autoradiographie pendant 24 à 48 heures.

Un criblage des colonies est effectué sur deux séries de filtres avec la sonde lapin. On obtient ainsi le clone 3.12 (figurer 3).

### 1.5. Synthèse d'ADNc grâce à l'oligonucléotide spécifique LPC₂ et insertion dans le vecteur pBluescript KS(+).

### 1.5.a. Synthèse d'ADNc grâce à l'oligonucléotide spécifique LPC₂.

Une synthèse d'ADNc simple brin est réalisée à partir de 5µg d'ARN polyA+, en présence de 50 unités d'AMV reverse transcriptase et de 100 ng d'un oligonucléotide synthétique LPC₂ spécifique de la LGC, et correspondant à la séquence en 3' de l'ADNc contenu dans le clone 3.12 précédemment décrit.

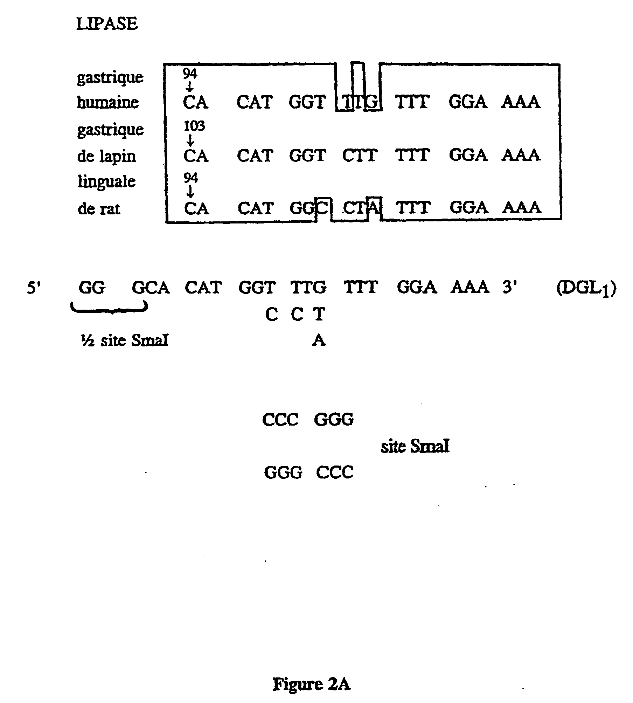

Après extraction de la solution au phénol chloroforme et précipitation dans l'alcool, le culot obtenu est dissous dans 20 microlitres d'eau distillée; l'ADNc simple brin en solution est alors amplifié et transformé en ADN double brin par la technique de la "PCR" à l'aide des amorces DGL₁ et LPC₂ présentées à la figure 2B, afin d'être cloné dans un vecteur approprié. L'amorce DGL₁ utilisée ci-dessus, est constituée d'un mélange de 12 séquences, chacune de ces séquences correspondant à l'une des combinaisons possibles de représentation de DGL₁ en tenant compte du fait que deux nucléotides T peuvent être remplacés par un nucléotide C, et qu'un nucléotide G peut être remplacé par un nucléotide T ou un nucléotide A, aux positions indiquées en dessous de l'amorce DGL₁ représentée sur les figures 2A et 2B.

### 1.5.b. Insertion dans le vecteur pBluescript KS(+).

Après digestion par l'enzyme PstI, le fragment de 700 pb d'ADNc est inséré dans le vecteur pBluescript KS(+) digéré par les enzymes de restriction SmaI et PstI.

Les molécules recombinantes issues de la ligation sont utilisées pour transformer des bactéries compétentes E.coli MM294. La sélection des transformants s'effectue en étalant le produit de la transformation sur un milieu nutritif solide (LB-Agar) contenant de l'ampicilline à 50 mg/litre.

On obtient ainsi le clone pKSPCR.

### 1.5.c. Ligation du fragment "H" du clone 3.12. dans le plasmide pKSPCR.

Le clone 3.12 est digéré par l'enzyme de restriction PstI; le fragment "H" PstI-PstI de 850 paires de bases du clone 3.12 correspondant à la région 3' de l'ADNc de la LGC, est inséré dans le plasmide pKSPCR préalablement linéarisé par l'enzyme PstI.

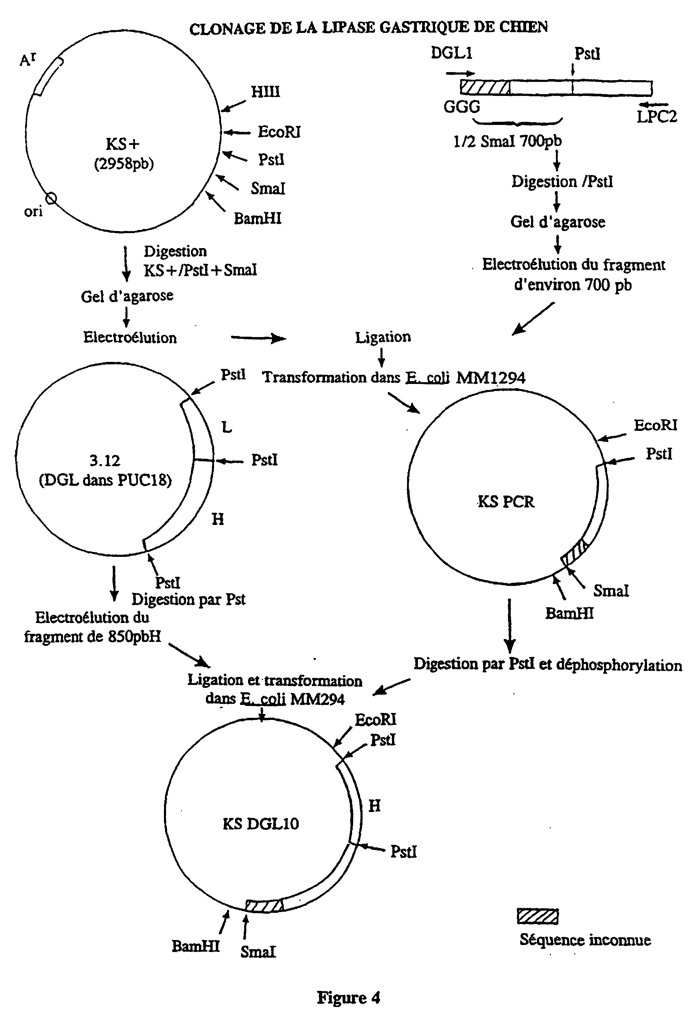

L'ensemble de ces étapes est présenté dans la figure 4.

### 1.6. Isolement de l'ADNc du clone pKS DGL10.

Un clone, pKS DGL10, a été sélectionné après le criblage par "PCR" (C. Blanchard et C. Benicourt, Boehringer, "Le brin complémentaire", septembre 1992, n°8, p6,). La coupure par des enzymes de restriction du plasmide pKSDGL10, montre qu'il contient un insert de 1,5 Kb. Ce plasmide est préparé à partir d'un litre de culture bactérienne en vue de son analyse détaillée et de son séquençage.

Le séquençage du clone est réalisé sur l'ADN double brin par la méthode de Sanger (Sanger, F. et al., Proc. Natl. Acad. Sci. USA. 74 (1977) 5463-5467).

La séquence complète de l'ADNc comporte 1528 nucléotides et est présentée dans la figure 8. Une phase ouverte de lecture allant du nucléotide 1 au nucléotide 1137 code pour une protéine de 379 AA. La séquence de cette protéine est rapportée dans la figure 9A. Cette protéine possède 81 % d'homologie avec la lipase gastrique de lapin (Brevet Français n° 91 13948).

### Etape n° 2: Construction de plasmides pour exprimer la LGC dans Escherichia Coli.

### 2.1. Choix du vecteur d'expression.

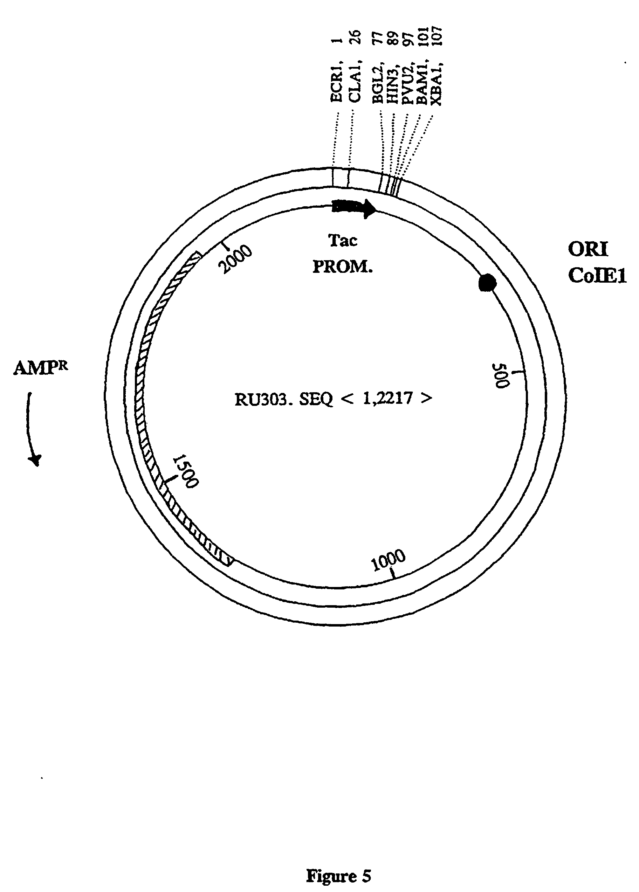

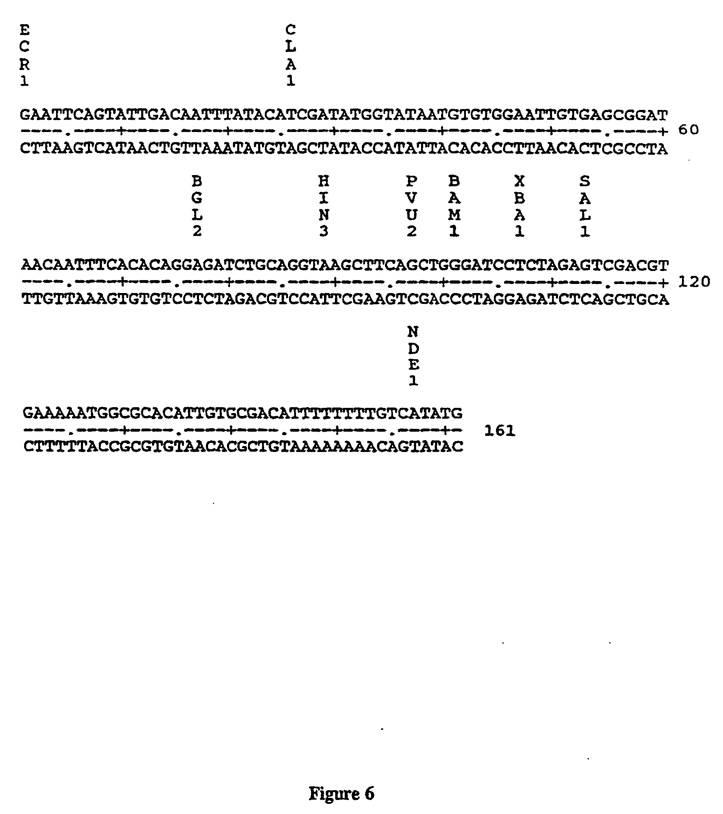

Le vecteur retenu pour exprimer la LGC dans E.coli est un plasmide dans lequel un fragment d'ADN synthétique de 160 pb contenant un promoteur de type Tac et un terminateur de transcription, a été inséré entre les sites EcoRI et NdeI de pBR322 (Bolivar, F. et al., Gene 2 (1977) 95-113). La carte de restriction du vecteur pRU303 est présentée dans la figure 5 et la séquence nucléotidique du fragment d' ADN EcoRI-NdeI dans la figure 6.

### 2.2. Construction du plasmide pDGL5.303

Malgré les études exhaustives qui ont été faites, peu de corrélations ont pu être établies entre le niveau d'expression d'une protéine hétérologue dans une bactérie et la séquence nucléotidique de la région 5'-terminale de l'ARN messager de cette même protéine. Cependant, nombre d'observations ont permis de déduire certaines règles empiriques pouvant conduire à un niveau d'expression plus élevé dans les bactéries recombinantes.

Parmi ces "règles", on peut citer:
- la distance entre la région de Shine et Dalgarno et l'AUG initiateur comprise entre 6 et 12 nucléotides,
- une séquence de Shine et Dalgarno riche en purines (AGGA),
- une structure secondaire minimale entre Shine et Dalgarno et l'AUG initiateur,
- l'absence de structure secondaire (double brin) dans les régions de l'ARN messager contenant la séquence Shine et Dalgarno et l'AUG initiateur.

On peut tenir compte de telles contraintes dans les programmes d'analyse permettant de définir les séquences nucléotidiques de régions 5' non codantes des ARNm susceptibles de conduire aux meilleurs niveaux d'expression de protéines hétérologues particulières, telles que la LGC dans E.coli.

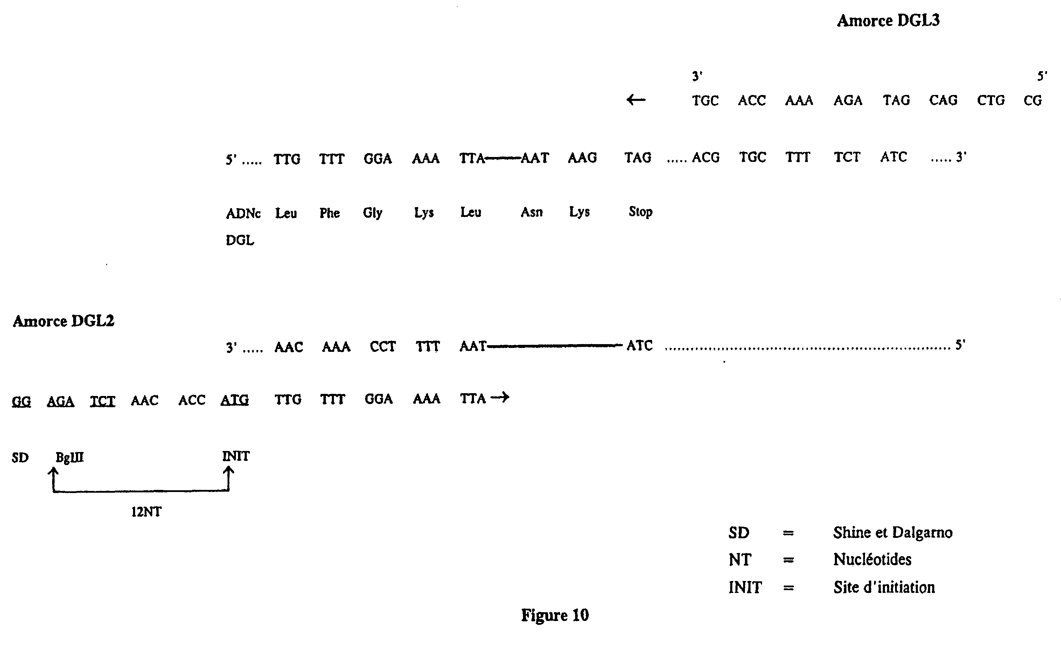

Grâce à des amorces synthétiques spécifiques DGL₂ et DGL₃ présentées dans la figure 10 et par la technique d'amplification génique "PCR", l'ADNc codant pour la partie mature de la LGC est positionné derrière un codon d'initiation de la traduction ATG et placé entre des séquences nucléotidiques telles qu'on puisse l'insérer dans le vecteur d'expression pRU303 entre les sites de restriction BglII et SalI. Du fait de la présence, dans la construction pDGL5.303, d'un codon ATG immédiatement en amont des séquences codant pour la LGC, les protéines recombinantes obtenues possèderont, totalement ou partiellement, une méthionine à leur extrémité NH2-terminale.

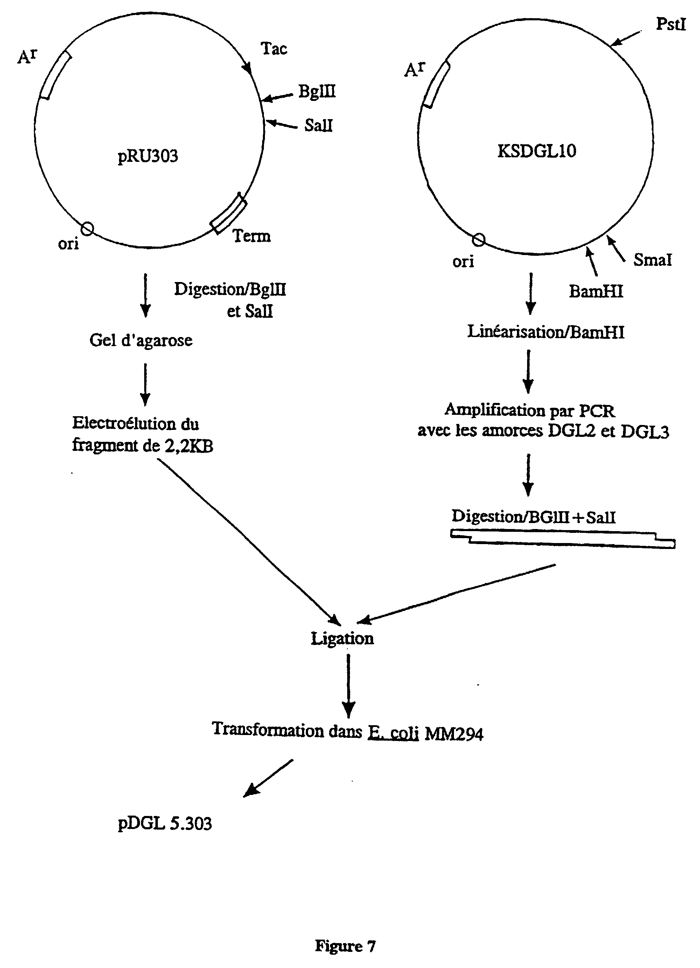

Le plasmide recombinant pDGL5.303 dont le schéma de construction est reporté dans la figure 7 a été obtenu dans la souche E.coli MM294 puis transféré dans la souche E.coli W3110 I^{q}, fréquemment utilisée pour l'expression de protéines hétérologues. Cette souche contient le gène du répresseur LacI^{q} situé sur un épisome non transférable F': le répresseur synthétisé en quantité importante dans la bactérie réprime l'expression de tous les gènes placés sous contrôle d'un promoteur de type lactose.

### Etape n°3: Expression de la LGC dans E. coli.

Le plasmide pDGL5.303 a été introduit dans l'hôte E.coli W3110 I^{q}. Les bactéries transformées par le plasmide sont cultivées dans du milieu en présence de M9 glucose (Maniatis, T. et al., déjà cité). Pendant la phase exponentielle de croissance, l'expression de la lipase gastrique de chien est induite par addition d'IPTG à la concentration finale de 2mM.

Après 4 heures à 37° C, les bactéries sont récoltées, centrifugées et lavées avec du tampon PBS. Les bactéries sont ensuite lysées dans un tampon contenant du SDS et du β-mercaptoéthanol pendant 10 minutes à 100° C.

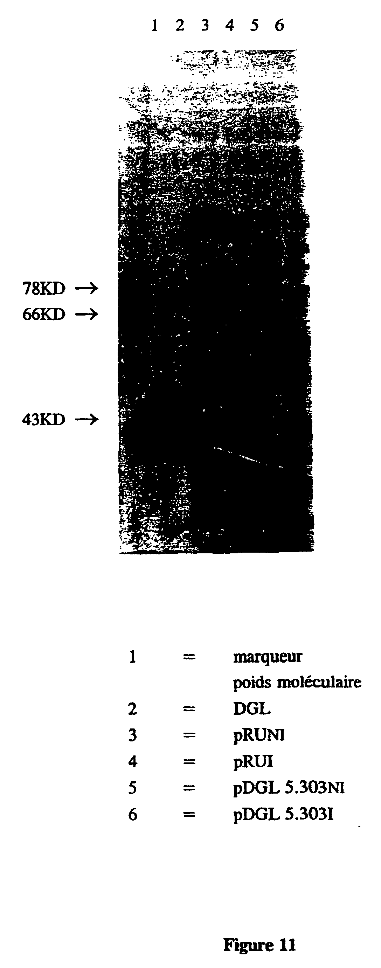

L'analyse des protéines sur gel d'électrophorèse en conditions dénaturantes permet la mise en évidence d'une bande protéique pouvant correspondre à la lipase. La protéine est exprimée à un taux tel qu'elle peut être détectée par cette technique comme ceci est montré dans la figure 11.

Afin de s'assurer que cette protéine, induite par addition d'IPTG au milieu de culture, correspond bien à la LGC, les protéines provenant de cultures de bactéries transformées par le plasmide pLGC5.303 induites et non-induites par l'agent chimique sont transférées sur une membrane de nylon après qu'elles aient été séparées selon leur taille par électrophorèse sur gel de polyacrylamide-SDS.

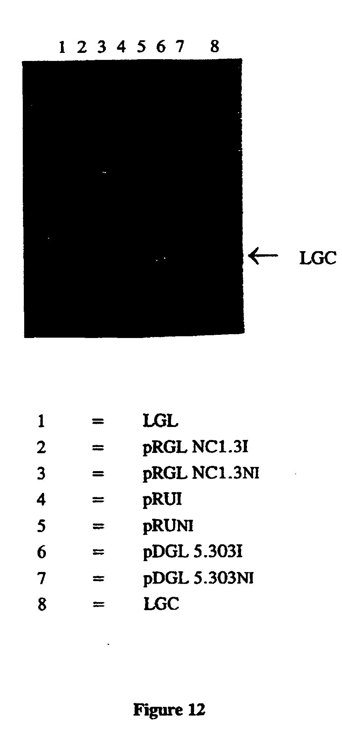

Le complexe entre la LGC et l'anticorps anti-LGC peut-être mis en évidence grâce à une réaction colorimétrique faisant intervenir un deuxième anticorps couplé à une enzyme, la peroxydase de raifort. Les résultats sont présentés dans la figure 12.

Plutôt que d'être produite sous forme de corps d'inclusion dans le cytoplasme de la bactérie *Escherichia coli*, la lipase gastrique de chien peut être avantageusement sécrétée dans le périplasme bactérien en insérant l'ADNc codant pour l'enzyme mature dans un vecteur approprié, sous contrôle d'un promoteur inductible par un agent physique ou chimique, et en aval d'un segment d'ADN codant pour un peptide signal tel que celui présent à l'extrémité NH₂-terminale de la protéine ompA (Movva N.R. et al. J. Biol. Chem. 256: 27-29, 1980).

La lipase gastrique de chien peut également être synthétisée dans *Escherichia coli* sous la forme d'une protéine de fusion soluble avec la protéine A de *Staphylococcus aureus* permettant sa purification ultérieure. A cette fin, l'ADNc codant pour la lipase mature est inséré dans le vecteur pRIT2T (Nilsson B. et al. EMBO J. 4: 1075-1080, 1985) qui a préalablement été modifié afin d'y introduire un fragment d'ADN codant pour le site de reconnaissance Ile-Glu-Gly-Arg du facteur Xa de la coagulation. La protéine de fusion ainsi produite peut être séparée des autres protéines du cytoplasme de la bactérie par chromatographie d'affinité sur une colonne d'IgG-Sépharose (Pharmacia). Après élution de la colonne, la protéine de fusion est clivée par le facteur Xa. Le produit de l'hydrolyse est à nouveau soumis à une chromatographie sur une colonne d'IgG-Sépharose qui retient la protéine A permettant ainsi d'obtenir la lipase gastrique de chien à l'état pur et sous forme soluble.

Il est également possible d'obtenir la lipase gastrique de chien à partir de cellules de mammifères en culture. Pour cela, il convient d'insérer l'ADNc codant pour le précurseur de cette lipase dans un vecteur approprié tel que le plasmide pCDNAI-Neo (Invitrogen corporation) sous contrôle du promoteur du Cytomégalovirus (CMV), ou encore l'ADNc codant pour la lipase mature dans le même type de vecteur, mais en aval d'un segment d'ADN codant pour le peptide signal de la lipase gastrique de lapin (Bénicourt C. et al; Demande de brevet Français n° 2 633 549 susmentionnée). L'introduction de tels plasmides recombinants dans les cellules COS-7 de rein de singe exprimant l'antigène T du virus SV40 de façon constitutive permet de produire transitoirement la lipase gastrique de chien dans le milieu de culture en quantité notable. Des lignées cellulaires exprimant la lipase gastrique de chien de façon constitutive peuvent être obtenues en introduisant l'un des deux plasmides recombinants dans des cellules d'ovaire de hamster (CHO) et en exerçant une pression de sélection avec l'antibiotique G418 ou généticine du fait de la présence d'un gène de résistance aux aminoglycosides tels que la néomycine sur lesdits plasmides.

La détection de l'activité de la LGC recombinante, notamment celle issue d'un lysat bactérien provenant d'une culture de W3110 I^{q} (pDGL5.303), est réalisée par la méthode de Gargouri et al (Gastroenterology 91 (1986) 265-275) en utilisant la tributyrine comme substrat.

On rappellera ci-après, les conditions expérimentales dans lesquelles les activités spécifiques des polypeptides recombinants, sont déterminés.

L'activité spécifique est définie comme étant le rapport de l'activité enzymatique et de la quantité de protéines de l'échantillon exprimée en milligrammes. L'activité lipasique est déterminée par la méthode titrimétrique de Y. Gargouri (préalablement cité), dans laquelle le substrat utilisé est la tributyrine. Le dosage consiste à neutraliser l'acide butyrique libéré sous l'action de la lipase par une solution de soude 0,1 N à pH constant de 6 et à une température de 37°C. Dans ces conditions d'essai, l'activité enzymatique correspond au nombre de micromoles d'acide libérées en une minute par l'action du produit soumis à l'essai.

Pratiquement, l'essai consiste à introduire, dans une cellule de titration thermostatée à 37°C:
- Tributyrine: 0,50 ml,
- Solution isotonique d'albumine bovine de sérum et de taurodésoxycholate de sodium 14,50 ml (Composition: albumine bovine de sérum 100 mg, taurodésoxycholate de sodium 2 mM, soluté isotonique à 0,9% de NaCl q.s.p. un litre).

Sous agitation électromagnétique et à l'aide d'un titrimètre automatique, le mélange est amené à pH 6 par addition de soude 0,1 N. Après stabilisation du pH à cette valeur, on ajoute, exactement mesurés, de 0,5 à 1 ml d'une solution aqueuse du composé enzymatique à doser. Dans ces conditions expérimentales, la quantité de solution de soude 0,1 N nécessaire pour maintenir le pH à 6 durant 2 minutes permet le calcul de l'activité lipasique telle que définie précédemment.

L'activité lipolytique peut également être mesurée par la méthode utilisant un substrat chromogénique tel que le 1,2-0-dilauryl-rac-glycéro-3-glutarate de résorufine (Boehringer), et décrite dans la notice du fabricant.

## Revendications

1. Acide nucléique **caractérisé en ce qu'**il comprend tout ou partie du fragment d'ADN représenté sur la figure 8, ledit acide nucléique codant pour le polypeptide délimité par les acides aminés situés aux positions 1 et 379 de la séquence en acides aminés représentée sur la figure 9A, ce polypeptide correspondant à la lipase gastrique de chien et possédant une activité lipolytique, ou codant pour un fragment de ce polypeptide, ce fragment conservant les propriétés enzymatiques du polypeptide susmentionné.

2. Acide nucléique selon la revendication 1, **caractérisé en ce qu'**il comprend tout ou partie du fragment d'ADN délimité par les nucléotides situés aux positions 1 et 1137 de l'ADN représenté sur la figure 8, et, le cas échéant, un fragment d'ADN codant pour une méthionine et situé en amont du fragment d'ADN susmentionné.

3. Acide nucléique **caractérisé en ce qu'**il comprend en amont d'un des fragments d'ADN selon la revendication 1, une séquence nucléotidique codant pour un peptide signal.

4. Acide nucléique simple brin **caractérisé en ce qu'**il comprend l'une ou l'autre des deux séquences nucléotidiques complémentaires constituant les fragments d'ADN selon l'une des revendications 1 à 3.

5. Acide nucléique codant pour le polypeptide délimité par les acides aminés situés aux positions 1 et 379 de la séquence en acides aminés représentée sur la figure 9a, ce polypeptide correspondant à la lipase gastrique de chien et possédant une activité lipolytique, ou codant pour un fragment dudit polypeptide conservant les propriétés enzymatiques dudit polypeptide, ledit acide nucléique étant **caractérisé en ce que** sa séquence nucléotidique digère, en fonction de la dégénérescence du code génétique, des séquences nucléotidiques définies dans l'une des revendications 1 à 4.

6. Acide nucléique recombinamt **caractérisé en ce qu'**il comprend un acide nucléique selon l'une des revendications 1 à 5, inséré dans une séquence nucléotidique hétérologue vis-à-vis du susdit acide nucléique.

7. Acide nucléique recombinant selon la revendication 6, **caractérisé en ce qu'**il comprend un promoteur situé en amont de l'acide nucléique selon l'une des revendications 1 à 5, et sous le contrôle duquel la transcription dudit acide nucléique est susceptible d'être effectuée, ainsi qu'une séquence codant pour des signaux de terminaison de la transcription située en aval dudit acide nucléique.

8. Vecteur recombinant, notamment du type plasmide, cosmide ou phage, **caractérisé en ce qu'**il contient un acide nucléique recombinant selon la revendication 6 ou la revendication 7, en l'un de ses sites non essentiels pour sa réplication.

9. Vecteur recombinant selon la revendication 8, **caractérisé en ce qu'**il contient en l'un de ses sites non essentiels pour sa réplication, des éléments nécessaires pour promouvoir et contrôler l'expression d'un acide nucléique selon l'une des revendications 1 à 5, dans un hôte cellulaire, et plus particulièrement un promoteur reconnu par les polymérases de l'hôte cellulaire, notamment un promoteur inductible.

10. Hôte cellulaire, de type procaryote ou eucaryote, transformé par un vecteur recombinant selon la revendication 8 ou la revendication 9, et comprenant les éléments de régulation permettant l'expression d'un acide nucléique selon l'une des revendications 1 à 7.

11. Polypeptide **caractérisé en ce qu'**il est codé par un acide nucléique selon l'une des revendications 1 à 5, et **en ce qu'**il possède une activité lipolytique, la LGC extraite et purifiée à partir d'estomacs de chien et présentant un poids moléculaire de 49 000 daltons étant exclue.

12. Polypeptide selon la revendication 11, correspondant selon le code génétique universel à l'acide nucléique selon la revendication 1, et délimité par les acides aminés situés aux positions 1 et 379 de la séquence en acides aminés représentée sur la figure 9A, ledit polypeptide possédant une activité lipolytique.

13. Polypeptide selon la revendication 11, correspondant selon le code génétique universel à l'acide nucléique selon la revendication 2, ce polypeptide étant constitué de la séquence en acides aminés délimitée par les acides aminés situés aux positions 1 et 379 de la figure 9A, et précédée d'une méthionine à l'extrémité NH2, ledit polypeptide possédant une activité lipolytique.

14. Polypeptide **caractérisé en ce qu'**il comprend un fragment des polypeptides recombinants selon l'une des revendications 11 à 13, ou tout polypeptide modifié par substitution et/ou addition et/ou suppression d'un ou plusieurs acides aminés de ces polypeptides recombinants, ces fragments ou polypeptides modifiés conservant les propriétés enzymatiques des polypeptides recombinants susmentionnés.

15. Procédé de préparation d'un polypeptide selon l'une des revendications 11 à 14, comprenant la succession d'étapes suivantes :
- la mise en culture d'un hôte cellulaire selon la revendication 10, dans un milieu de culture approprié, et
- la récupération du polypeptide produit par ledit hôte cellulaire, soit directement à partir du susdit milieu de culture, soit après lyse de l'hôte cellulaire.

16. Composition pharmaceutique **caractérisée en ce qu'**elle comprend au moins un type de polypeptide selon l'une des revendications 11 à 14, le cas échéant en combinaison avec un ou plusieurs autres polypeptides à activité lipasique, en association avec un véhicule pharmaceutiquement acceptable.

17. Utilisation d'un ou plusieurs des polypeptides selon l'une des revendications 11 à 14, pour l'obtention de médicaments destinés à faciliter l'absorption des graisses animales ou végétales ingérées par un individu sain ou atteint d'une ou plusieurs pathologies affectant ou non le taux de production de lipase gastrique.

18. Utilisation d'un ou plusieurs des polypeptides selon l'une des revendications 11 à 14, pour l'obtention de médicaments destinés au traitement des pathologies liées à l'insuffisance, voire l'absence, de production de lipases dans l'organisme, et plus particulièrement de pathologies telles que la mucoviscidose, et l'insuffisance pancréatique exocrine.

19. Utilisation d'un ou plusieurs des polypeptides selon l'une des revendications 11 à 14, pour la mise en oeuvre de réactions de bioconversions enzymatiques (telles que hydrolyses, trans-estérifications enzymatiques), notamment sous forme immobilisée sur un support solide.

## Patentansprüche

1. Nukleinsäure,
**dadurch gekennzeichnet, dass** sie die Gesamtheit oder einen Teil des in Figur 8 dargestellten DNA-Fragments umfasst, wobei die Nukleinsäure für das Polypeptid codiert, das durch die in den Positionen 1 und 379 der in Figur 9A dargestellten Aminosäuresequenz befindlichen Aminosäuren begrenzt ist, wobei dieses Polypeptid der Hundegastrolipase entspricht und eine lipolytische oder für ein Fragment dieses Polypeptids codierende Aktivität besitzt, wobei dieses Fragment die enzymatischen Eigenschaften des oben erwähnten Polypeptids bewahrt.

2. Nukleinsäure nach Anspruch 1,
**dadurch gekennzeichnet, dass** sie die Gesamtheit oder einen Teil des DNA-Fragments, das von den in den Positionen 1 und 1137 der in Figur 8 dargestellten DNA befindlichen Nukleotiden begrenzt ist, und gegebenenfalls ein für ein Methionin codierendes und stromaufwärts zu dem oben erwähnten DNA-Fragment liegendes DNA-Fragment umfasst.

3. Nukleinsäure,
**dadurch gekennzeichnet, dass** sie stromaufwärts zu einem der DNA-Fragmente nach Anspruch 1 eine für ein Signalpeptid codierende Nukleotidsequenz umfasst.

4. Einsträngige Nukleinsäure,
**dadurch gekennzeichnet, dass** sie die eine oder die andere der beiden komplementären Nukleotidsequenzen umfasst, die die DNA-Fragmente nach einem der Ansprüche 1 bis 3 darstellen.

5. Nukleinsäure, die für das Polypeptid codiert, das von den in den Positionen 1 und 379 der in Figur 9A dargestellten Aminosäuresequenz befindlichen Aminosäuren begrenzt ist, wobei dieses Polypeptid der Hundegastrolipase entspricht und eine lipolytische Aktivität besitzt, oder für ein Fragment des Polypeptids codiert, das die enzymatischen Eigenschaften des Polypeptids bewahrt, wobei die Nukleinsäure **dadurch gekennzeichnet ist, dass** sich ihre Nukleotidsequenz in Abhängigkeit von der Degeneration des genetischen Codes von den Nukleotidsequenzen unterscheidet, die in einem der Ansprüche 1 bis 4 definiert sind.

6. Rekombinante Nukleinsäure,
**dadurch gekennzeichnet, dass** sie eine Nukleinsäure nach einem der Ansprüche 1 bis 5 umfasst, die in eine gegenüber der Nukleinsäure heterologe Nukleotidsequenz inseriert ist.

7. Rekombinante Nukleinsäure nach Anspruch 6,
**dadurch gekennzeichnet, dass** sie einen Promotor umfasst, der stromaufwärts zu der Nukleinsäure nach einem der Ansprüche 1 bis 5 angeordnet ist, und unter dessen Kontrolle die Transkription der Nukleinsäure stattfinden kann, sowie eine für Signale zur Termination der Transkription codierende Sequenz, die stromabwärts zu der Nukleinsäure angeordnet ist.

8. Rekombinanter Vektor, insbesondere ein Plasmid, Cosmid oder Phage,
**dadurch gekennzeichnet, dass** er eine rekombinante Nukleinsäure nach Anspruch 6 oder Anspruch 7 an einer seiner nicht für seine Replikation notwendigen Stellen enthält.

9. Rekombinanter Vektor gemäß Anspruch 8,
**dadurch gekennzeichnet, dass** er an einer seiner nicht für seine Replikation notwendigen Stellen Elemente enthält, die notwendig sind, um die Expression einer Nukleinsäure nach einem der Ansprüche 1 bis 5 in einer Wirtszelle zu fördern und zu kontrollieren, und insbesondere einen Promotor, der von den Polymerasen der Wirtszelle erkannt wird, insbesondere einen induzierbaren Promotor.

10. Prokaryote oder eukaryote Wirtszelle, die mit einem rekombinanten Vektor nach Anspruch 8 oder Anspruch 9 transformiert wurde und Regulationselemente umfasst, die die Expression einer Nukleinsäure nach einem der Ansprüche 1 bis 7 ermöglichen.

11. Polypeptid,
**dadurch gekennzeichnet, dass** es durch eine Nukleinsäure nach einem der Ansprüche 1 bis 5 codiert wird, und dass es eine lipolytische Aktivität besitzt, wobei die Hundegastrolipase, die aus Hundemägen extrahiert und gereinigt wird und ein Molekulargewicht von 49 000 Dalton aufweist, ausgeschlossen ist.

12. Polypeptid nach Anspruch 11, das nach dem universellen genetischen Code der Nukleinsäure nach Anspruch 1 entspricht und von den in den Positionen 1 und 379 der in Figur 9A dargestellten Aminosäuresequenz befindlichen Aminosäuren begrenzt ist, wobei das Polypeptid eine lipolytische Aktivität aufweist.

13. Polypeptid nach Anspruch 11, das nach dem universellen genetischen Code der Nukleinsäure nach Anspruch 2 entspricht, wobei dieses Polypeptid von der Aminosäuresequenz gebildet ist, die von den in den Positionen 1 und 379 der Figur 9A befindlichen Aminosäuren begrenzt ist und der ein Methionin am NH₂-Terminus vorangestellt ist, wobei das Polypeptid eine lipolytische Aktivität besitzt.

14. Polypeptid,
**dadurch gekennzeichnet, dass** es ein Fragment der rekombinanten Polypeptide nach einem der Ansprüche 11 bis 13 oder ein durch Substitution und/oder Addition und/oder Deletion einer oder mehrerer Aminosäuren dieser rekombinanten Polypeptide modifiziertes Polypeptid umfasst, wobei diese Fragmente oder modifizierten Polypeptide die enzymatischen Eigenschaften der oben erwähnten rekombinanten Polypeptide bewahren.

15. Verfahren zur Herstellung eines Polypeptids nach einem der Ansprüche 11 bis 14, das nacheinander die folgenden Schritte umfasst:
- Kultivieren einer Wirtszelle nach Anspruch 10 in einem geeigneten Kulturmedium, und
- Wiedergewinnung des von der Wirtszelle produzierten Polypeptids entweder direkt aus dem oben erwähnten Kulturmedium oder nach Lyse der Wirtszelle.

16. Pharmazeutische Zusammensetzung,
**dadurch gekennzeichnet, dass** sie mindestens ein Polypeptid nach einem der Ansprüche 11 bis 14, gegebenenfalls in Kombination mit einem oder mehreren anderen Polypeptiden mit Lipaseaktivität, in Verbindung mit einem pharmazeutisch annehmbaren Träger umfasst.

17. Verwendung eines oder mehrerer Polypeptide nach einem der Ansprüche 11 bis 14 zur Gewinnung von Medikamenten, die dazu bestimmt sind, die Absorption von tierischen oder pflanzlichen Fetten, die von einem gesunden oder einem von einer oder mehreren, die Gastrolipaseproduktion beeinträchtigenden oder nicht beeinträchtigenden Pathologien betroffenen Individuum aufgenommen werden, zu erleichtern.

18. Verwendung eines oder mehrerer Polypeptide nach einem der Ansprüche 11 bis 14 zur Gewinnung von Medikameten, die für die Behandlung von Pathologien bestimmt sind, die mit der Insuffizienz oder dem Fehlen einer Lipaseproduktion im Organismus verbunden sind, und insbesondere von Pathologien, wie beispielsweise der Mukoviszidose und der exokrinen Pankreasinsuffizienz.

19. Verwendung eines oder mehrerer Polypeptide nach einem der Ansprüche 11 bis 14 für den Einsatz von enzymatischen Bioumwandlungsreaktionen (wie beispielsweise Hydrolysen, enzymatische Trans-Veresterungen), insbesondere in auf einem festen Träger immobilisierter Form.

## Claims

1. Nucleic acid, **characterized in that** it comprises all or part of the DNA fragment shown in Figure 8, said nucleic acid coding for the polypeptide bounded by the amino acids located in positions 1 and 379 of the amino acid sequence shown in Figure 9A, this polypeptide corresponding to canine gastric lipase and possessing a lipolytic activity, or coding for a fragment of this polypeptide, this fragment preserving the enzymatic properties of the above-mentioned polypeptide.

2. Nucleic acid according to Claim 1, **characterized in that** it comprises all or part of the DNA fragment bounded by the nucleotides located in positions 1 and 1137 of the DNA shown in Figure 8, and, if appropriate, a DNA fragment coding for a methionine and located upstream from the above-mentioned DNA fragment.

3. Nucleic acid, **characterized in that** it comprises, upstream from one of the DNA fragments according to Claim 1, a nucleotide sequence coding for a signal peptide.

4. Single-stranded nucleic acid, **characterized in that** it comprises one or other of the two complementary nucleotide sequences constituting the DNA fragments according to one of Claims 1 to 3.

5. Nucleic acid coding for the polypeptide bounded by the amino acids located in positions 1 and 379 of the amino acid sequence shown in Figure 9A, this polypeptide corresponding to canine gastric lipase and possessing a lipolytic activity, or coding for a fragment of said polypeptide preserving the enzymatic properties of said polypeptide, said nucleic acid being **characterized in that** its nucleotide sequence differs, according to the degeneracy of the genetic code, from the nucleotide sequences defined in one of Claims 1 to 4.

6. Recombinant nucleic acid, **characterized in that** it comprises a nucleic acid according to one of Claims 1 to 5 inserted in a nucleotide sequence heterologous to the above-mentioned nucleic acid.

7. Recombinant nucleic acid according to Claim 6, **characterized in that** it comprises a promoter located upstream from the nucleic acid according to one of Claims 1 to 5, and under whose control said nucleic acid is capable of being transcribed, as well as a sequence coding for transcription termination signals and located downstream from said nucleic acid.

8. Recombinant vector, especially of the plasmid, cosmid or phage type, **characterized in that** it contains a recombinant nucleic acid according to Claim 6 or Claim 7 at one of its sites not essential for its replication.

9. Recombinant vector according to Claim 8, **characterized in that** it contains, at one of its sites not essential for its replication, components necessary for promoting and controlling the expression of a nucleic acid according to one of Claims 1 to 5 in a cellular host, and more particularly a promoter recognized by the polymerases of the cellular host, especially an inducible promoter.

10. Cellular host, of the prokaryotic or eukaryotic type, transformed by a recombinant vector according to Claim 8 or Claim 9 and comprising the regulatory components allowing the expression of a nucleic acid according to one of Claims 1 to 7.

11. Polypeptide, **characterized in that** it is encoded by a nucleic acid according to one of Claims 1 to 5 and **in that** it possesses a lipolytic activity, with the exclusion of the CGL extracted and purified from dog stomachs and having a molecular weight of 49,000 daltons.

12. Polypeptide according to Claim 11 which corresponds, according to the universal genetic code, to the nucleic acid according to Claim 1 and is bounded by the amino acids located in positions 1 and 379 of the amino acid sequence shown in Figure 9A, said polypeptide possessing a lipolytic activity.

13. Polypeptide according to Claim 11 which corresponds, according to the universal genetic code, to the nucleic acid according to Claim 2, this polypeptide consisting of the amino acid sequence bounded by the amino acids located in positions 1 and 379 of Figure 9A and preceded by a methionine at the NH₂ end, said polypeptide possessing a lipolytic activity.

14. Polypeptide, **characterized in that** it comprises a fragment of the recombinant polypeptides according to one of Claims 11 to 13, or any polypeptide modified by the substitution and/or addition and/or elimination of one or more amino acids of these recombinant polypeptides, these fragments or modified polypeptides preserving the enzymatic properties of the above-mentioned recombinant polypeptides.

15. Process for the preparation of a polypeptide according to one of Claims 11 to 14 which comprises the following series of steps:
- the cultivation of a cellular host according to Claim 10 in an appropriate culture medium, and
- the recovery of the polypeptide produced by said cellular host, either directly from the above-mentioned culture medium or after lysis of the cellular host.

16. Pharmaceutical composition, **characterized in that** it comprises at least one type of polypeptide according to one of Claims 11 to 14, if appropriate in combination with one or more other polypeptides with lipase activity, in association with a pharmaceutically acceptable vehicle.

17. Use of one or more of the polypeptides according to one of Claims 11 to 14 for the preparation of drugs for facilitating the absorption of animal or vegetable fats ingested by a healthy individual or an individual suffering from one or more pathological conditions that may or may not affect the rate of production of gastric lipase.

18. Use of one or more of the polypeptides according to one of Claims 11 to 14 for the preparation of drugs for the treatment of pathological conditions associated with the insufficient or even non-existent production of lipases in the organism, and more particularly of pathological conditions such as cystic fibrosis and exocrine pancreatic insufficiency.

19. Use of one or more of the polypeptides according to one of Claims 11 to 14 for carrying out enzymatic bioconversion reactions (such as enzymatic hydrolyses and transesterifications), especially in a form immobilized on a solid support.
